(19) Europäisches Patentamt
European Patent Office
Office européen des brevets

(11) **EP 3 425 542 A1**

(12) **EUROPEAN PATENT APPLICATION**
published in accordance with Art. 153(4) EPC

(43) Date of publication:
**09.01.2019 Bulletin 2019/02**

(51) Int Cl.:
**G06F 19/00** *(2018.01)*    **G08B 21/02** *(2006.01)*

(21) Application number: **17759307.6**

(22) Date of filing: **03.01.2017**

(86) International application number:
**PCT/ES2017/070004**

(87) International publication number:
**WO 2017/149174 (08.09.2017 Gazette 2017/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.02.2016 ES 201600158**

(71) Applicants:
• **Fundación Para La Investigación Biomédica Del Hospital De La Princesa**
**28006 Madrid (ES)**
• **Universidad Complutense De Madrid**
**28015 Madrid (ES)**

(72) Inventors:
• **GAGO VEIGA, Ana Beatriz**
**28006 Madrid (ES)**
• **SOBRADO SANZ, Mónica**
**28006 Madrid (ES)**
• **VIVANCOS MORA, Jose Aurelio**
**28006 Madrid (ES)**
• **PAGÁN ORTIZ, Josué**
**28015 Madrid (ES)**
• **DE ORBE IZQUIERDO, María Irene**
**28015 Madrid (ES)**
• **AYALA RODRIGO, José Luis**
**28015 Madrid (ES)**

(74) Representative: **Temiño Ceniceros, Ignacio**
**Abril Abogados**
**Amador de los Rios 1-1°**
**28010 Madrid (ES)**

(54) **METHOD FOR DETERMINING THE DEGREE OF ACTIVATION OF THE TRIGEMINOVASCULAR SYSTEM**

(57)    The present invention describes a method for determining in real time the level of activation of the trigeminovascular system. In particular, said invention can be applied in the field of medical devices capable of determining the activation index of the trigeminovascular system, mainly on the basis of the use of biomedical signals of hemodynamic character. The method establishes objective criteria for determining the degree of activation and is described as the result of the application of modelling and data fusion techniques. The method is also based on another type of signals, such as ambient signals, in order to improve statistically in real time the degree of activation determined.

*Figure 2*

**Description**

**TECHNICAL FIELD**

The present invention mainly falls within the field of medical devices. In particular, it is focused on modelling and prediction systems applied to neurological diseases and, more specifically, the prediction of migraine attacks with the goal of becoming more effective at reducing pain with pharmacological treatments.

**STATE OF THE ART**

Migraines are a type of headache that is characterised by a pain with a number of characteristics defined by: duration between 4 and 72 hours, moderate-severe intensity, worsens with exercise, throbbing, interrupts the activity of the patient and is usually accompanied by symptoms such as nausea, vomiting, photophobia and/or phonophobia. Today, migraines are a very significant public health problem due to the high prevalence thereof which brings with it a significant burden for patients, families and society. Migraines are considered one of the most debilitating pathologies with significant consequences on social and work activities (absenteeism and reduced productivity at work) and, therefore, a high socioeconomic cost (Linde M, Rastenyte D, Ruiz de la Torre E, et al. The cost of headache disorders in Europe: The Eurolight project. European journal of neurology: the official journal of the European Federation of Neurological Societies. 2012; 19(5):703-711*)*.

In treating migraine pain, it is known that early treatment is much more effective, since once the central sensitisation of the trigeminal nerve is produced, within the pain cycle, it is much more complicated to stop. Thus, the fundamental objective in the clinical practise is to treat the pain before this central sensitisation takes place. Consequently, it is recommended that the patient take analgesics as soon as the pain begins, with the aim of stopping the migraine attack, since if the medication is taken too late, it tends to not be effective.

Published studies show that the dopaminergic antagonist, domperidone *(*Waelkens J. Dopamine blockade with domperidone: Bridge between prophylactic and abortive treatment of migraine? A dose-finding study. Cephalalgia. 1984;4(2):85-90*).,* and triptan, naratriptan (Luciani R, Carter D, Mannix L, Hemphill M, Diamond M, Cady R. Prevention of migraine during prodrome with naratriptan. Cephalalgia. 2000; 20(2):122-126*),* drugs used in the acute phase, can make the pain go away when they are administered early, in the prodrome. The prediction based on prodromal symptoms by patients themselves can have a number of limitations, since the prodromal symptoms have a variable time horizon, meaning, the patient does not know exactly when the pain will occur, the symptoms are very unspecific (changes in mood, appetite, sleep pattern, etc.), they can happen to them in any other situation and they are not very precise *(*Becker WJ. The premonitory phase of migraine and migraine management. Cephalalgia. 2013; 33(13):1117-1121*;* Rossi P, Ambrosini A, Buzzi MG. Prodromes and predictors of migraine attack. Funct Neurol. 2005; 20(4):185-191*;* Giffin NJ, Ruggiero L, Lipton RB, et al. Premonitory symptoms in migraine: An electronic diary study. Neurology. 2003; 60(6):935-940).Thus, we may be less effective when reducing pain or deciding to take a medicinal product when a migraine is not going to occur.

For this reason, by objectively knowing that when the patient is going to have pain before they suffer from it (prediction), it will be possible to not only provide treatment that is early but also one that is better targeted; by knowing when the pain could occur and, knowing the action mechanism of the drug, the most convenient treatment for the patient can be chosen, since by knowing when the pain will occur, the action mechanism of the drug can be seen and the most convenient treatment can be considered. The method proposed in the present invention enables the pain of the patient to be predicted and the treatment to be more effective.

In the state of the art, the involvement of the autonomic nervous system (ANS) in the migraine is notable, demonstrating that there is a dysautonomic dysfunction, which manifests as an alteration of the variables controlled by it in the migraine patient and that it is involved both in the genesis and in the persistence of the migraine.

There are still many unknowns regarding the nature of said dysautonomia of the migraine patient, in particular, it is unknown if it is the cause or consequence of it, considering it simply as an epiphenomenon. The clinical studies that evaluate the autonomic function in patients with migraines have showed differing results (Gass JJ, Glaros AG. Autonomic dysregulation in headache patients. Appl Psychophysiol Biofeedback. 2013; 38(4):257-263*;* Mosek A, Novak V, Opfer-Gehrking TL, Swanson JW, Low PA. Autonomic dysfunction in migraineurs. Headache: The Journal of Head and Face Pain. 1999;39(2):108-117*;* Sanya E, Brown C, Wilmowsky C, Neundörfer B, Hilz M. Impairment of parasympathetic baroreflex responses in migraine patients. Acta Neurol Scand. 2005;111(2):102-107 and Benjelloun H, Birouk N, Slaoui I, et al. Autonomic profile of patients with migraine. Neurophysiol Clin. 2005; 35(4):127-134.*),* which describe a hypofunction and hyperfunction of both the sympathetic and parasympathetic systems.

Only some studies have evaluated the changes between the baseline situation and the symptomatic period in the migraine patient, among which are notable: Duru M, Melek I, Seyfeli E, et al. QTc dispersion and p-wave dispersion during migraine attacks. Cephalalgia. 2006;26(6): 672-677*,* which shows the association in the migraine attack with an increase of QTc interval of the electrocardiogram signal and dispersion of the P-wave in pain-free periods, the studies performed by Ordás CM, Cuadrado ML, Rodriguez-Cambron AB, Casas-Limón J, del Prado N, Porta-Etessam J. Increase in body temperature during migraine attacks. Pain Medicine. 2013; 14(8):1260-1264 *y* Porta-Etessam J, Cuadrado ML, Rodriguez-Gomez O, Valencia C, Garcia-Ptacek S. Hypothermia during migraine attacks. Cephalalgia. 2010; 30(11):1406-1407. also being notable, where a case of a patient with hypothermia and another with hyperthermia during the pain period is described. In a study by Dr. Seçil (*Seçil Y, Ünde C, Beckmann YY, Bozkaya YT, Özerkan F, BaŞoǦlu M. Blood pressure changes in migraine patients before, during and after migraine attacks. Pain practice. 2010; 10(3):222-227*) a tendency towards diastolic hypertension was observed. In relation to the state of the art relating to the changes that occur at an electroencephalographic level the studies by Bjork are notable (Bjørk M, Sand T. Quantitative EEG power and asymmetry increase 36 h before a migraine attack. Cephalalgia. 2008; 28(9):960-968*)* which demonstrate a slower and more asymmetrical activity before the start of the pain also in relation to the duration of the attack and the intensity.

Nevertheless, none of these studies present the results for continuous outpatient monitoring in real time during the pre-migraine period, the migraine phase and the post-migraine period, nor do they apply pain prediction algorithms.

Meanwhile the patent application WO03063684 (Geatz M, Roiger R.) proposes the use of a system and a method for predicting the symptoms through physiological variables with applications to diseases of different natures, one of these being migraines. However, the proposed methodology has a high level of abstraction, not technically motivating the selection of variables for a certain pathology.

Other patent applications also describe, but in a more detailed manner, closed-loop control systems with applications for, in this case, diabetes (ES2334733) and epilepsy (US2011270095). However, none of these propose adaptive techniques when faced with data loss in order to make a robust system.

The application TW201023087 mentions the use of a network of sensors in order to predict, apart from not mentioning the industrial application, the claims are based on the efficient consumption and on the handling of the data acquisition through knowledge of the state of the prediction.

Moreover, the patent US8123683, which refers to headaches in general, has the goal of detecting the type of headache produced; this is achieved by grouping different triggers that the patient can indicate to the system. Nevertheless, said document only claims the classification of the attack and not the prediction thereof.

In the industrial field, several mobile applications have been developed that act as calendars and classifiers for migraines (Migraine Buddy, My Migraine Triggers™ and My Migraines); nevertheless, none of these is used to predict attacks in real time, and they do not perform outpatient monitoring of biometric variables.

In conclusion, none of these documents propose:

1. A method for predicting migraine attacks through non-invasive outpatient monitoring of biometric variables and the registration of environmental variables.
2. A detailed prediction methodology and a selection of the variables to be monitored that is justified and based on medical literature.
3. A robust method when facing partial or total data loss and sensor saturation, by means of a Sensor-dependent Model Selection System (SDMS[2]).
4. The linear combination of migraine models through the selection of several models trained for each patient.
5. A system that implements a hierarchical module for selecting models for each patient depending on the availability of the sensors.

A system that implements an expert prediction-improving module for eliminating false detections that could be considered as false positives.

Preliminary results performed by the authors of the present invention confirm the following technological advantages:

1. Prediction of the neurological pathology of the migraine through variables controlled by the autonomous nervous system in a non-intrusive manner.
2. Fulfilment of prediction time horizons within the delay in actuation of the medicinal products against the migraine pain so that taking them is more effective.
3. Definition of a system that is robust against data losses and errors such that it is able to maintain a certain prediction horizon.
4. Creation of a prediction as a consequence of the linear combination of the result from several models with the aim of reducing the variability of the predictions and provide them with reliability.
5. Use of an expert prediction-improving module that is able to practically eliminate false alarms from the entirety of the prediction.

**DETAILED DESCRIPTION OF THE INVENTION**

The invention has the object of predicting migraine attacks in real time through a prediction method that is robust and adaptive with regards to data and/or sensor losses, and which uses non-invasive outpatient monitoring of biometric variables of the patient and environmental variables. To do so, a hierarchical system of prediction models according to the set of sensors available at all times is defined, adapted to each patient.

The invention is made up of a method for predicting migraines in real time.

The method is structured in three steps (Data acquisition, Training and validation of the migraine models and Prediction in real time) which in turn are subdivided into different modules.

In the Data acquisition step, the biometric (d1) and environmental (d5) variables, the subjective sensation of pain of the patient during the migraine (d2), information about the activity of the patient that could affect the migraine episodes (d3) and the clinical data of the patient (d4) are recorded. The set of this data is transmitted from the monitoring equipment to cloud servers. Said low-cost equipment for monitoring biometric variables has batteries with a limited duration; nevertheless, they have enough processing ability so that, by being aware of the state of the prediction, they are able to decide when to temporarily pause the outpatient monitoring and thus reduce the consumption of the batteries thereof.

The Training and Validation step of the models is carried out in servers, which is external equipment with large computational capacities. The data (d1 and d2) is used, in a first instance, for the creation of migraine models personalised for each patient that will be used in real time in the third step, for the prediction of the migraine attack. This last step, Prediction in real time of the migraine attack, is performed in a feedback loop starting from the input data (d1, d3-d5). The prediction based on environmental data (d5), activity data (d3) and clinical data (d4) will be used in the module of the decision system (7).

The steps to be followed in each step and the way in which each module intervenes are described below.

**A) Data acquisition**

The method for predicting a migraine attack that is presented in the invention implies the use of the following multisource data:

- Biometric data from the patients (d1): hemodynamic signals and cerebral electrical activity. The biometric data from the patients is collected through a low-cost wireless body sensor network (or WBSN). Said device is non-invasive and collects the following variables: the electrocardiogram ECG and the electrodermal activity or galvanic skin response (EDA or GSR, h1) by means of superficial electrodes; the superficial temperature of the skin (h2) through a thermistor; the oxygen saturation in blood (SpO2, h3) and the photoplethysmography curve (PPG, h4) by means of an infrared reflective sensor. The secondary variables used as inputs for the system are obtained in the module for pre-processing the signal (1). The heart rate (HR, hs1) is calculated starting from the ECG signal. Likewise, the pulse transit time (PTT, hs2) is obtained through the combination of the ECG and PPG variables. The cerebral electrical activity electroencephalography (EEG, h5) is measured at the occipital level. Using this activity, the power bands of the brain waves (qEEG, hs3) are quantified. The choice of these variables is made according to the alteration thereof by the autonomous nervous system, mentioned above.
- Climatological data from the geographical area in which the patient is found and local environmental data (d5): the climatological data from the geographical area are collected from a government weather service, and are: temperature (a1), relative humidity (a2), atmospheric pressure (a3) and precipitation (a4). The local measurements are performed through a meteorological station always close to the patient connected to a mobile phone, and they include: temperature of the room (al1), relative humidity (al2), pressure (al3), light (al4) and sound pressure level (al5).
- Subjective sensation of pain of the patient in each migraine episode (d2): the patient indicates the start and end of the pain, as well as the prodromal symptoms and/or auras that they may suffer from in the data-collecting application of a smartphone. The subjective evolution of the pain is also recorded as relative increases and decreases in pain, drawing a curve that reflects the levels of pain intensity that the patient feels in different moments of the migraine.
- The information relating to the activity of the patient (d3) that may be relevant for the study of the migraine will play a significant role in the method. The information is collected in an application that runs on a smartphone and refers to the ingestion of food (such as: dairy, fruit, alcohol, etc.), physical or mental activity, mood, ingestion of medicinal products or subjective sensations such as prodromal symptoms or auras.
- The clinical information of the patient (d4) is also taken into account. Gender, age, related diseases or prescribed medication can be relevant information in the prediction process. This sensitive information is anonymous and is collected at the beginning of the study.

**a) Pre-processing modules of the signal (1, 1-2 and 2).**

The data gathered by the wireless monitoring systems can suffer losses due to the cessation of wireless communication or to sensor failures. In order to repair the signal losses in these time intervals, signal regeneration techniques are applied in the module (1) for automatic signal regeneration based on the statistical behaviour thereof. Before repair, the calculation of the secondary variables (hs1-3) is also performed in this module. In this module, apart from calculating the secondary variables and repairing lost data, the synchronisation of the data is also performed in order to adjust the sampling rates of the different signals. The result from the module (1) is the signal (b), which has two types of different signals, those of biometric origin (b[1]) and those of environmental origin (b[2]). Thus, $b = b[1] \cup b[2]$.

The module (1-2) is responsible for calculating derived variables (c) and extracting knowledge by means of Machine Learning techniques and is executed parallel to the module (1). The derived variables are features of the primary biometric variables (al1-5) and secondary biometric variables (hs1-3) and of the overall environmental variables (a1-4) and local environmental variables (al1-5). These chosen features can be different for each patient, and the study of which ones are better is automated for each of them. These features can be temporal variables (hd(t)) or specific values (figures of merit, hD). The most common features that are looked for in these signals are: energy from the signal, energy in a band of interest of the signal, moving average, maximum, minimum and average values, etc. The features that vary in time $hd = \{hd1(t), h2(t),...,hdH(t)\}$, will be used in the module (3) for training models. Note that, although no explicit reference is made, the derived signals are the signal (c) and they include those of biometric origin (c[1]) and those of environmental origin (c[2]). Thus, $c = c[1] \cup c[2]$. The features (hD) are calculated and they use the module which, together with the activity data (d3) and clinical data (d4) of the patient, give rise to the signal (g). The signal (g) is the result of applying fuzzy logic techniques, which will provide classification criteria to help improve the prediction of the expert system (7).

Signal regeneration and the calculation of derived variables are also applied to the environmental data (d5). Like the biometric variables, migraine prediction models will be trained with the repaired environmental data (b[2]) and with derived environmental data (c[2]).

These modules are very important since the validity of the obtained models (d) depends on the quality of the data.

In order to calculate the models, the output signal is needed, which is the pain signal. The supervision module of the pain (2) consists of reading the recordings of the subjective pain of the patient. This module generates a synthetic curve of the pain by means of a bilateral Gaussian fit of the pain evolution points marked by the patient (d2). This evolution is recorded as whole values without upper or lower limits, which represent relative changes with respect to the last moment marked. This unlimited scale, compared to others that are limited, enables a more accurate curve of the evolution of the pain of the patient to be generated. Given that they do not know a priori when the maximum pain thereof is reached or how much it is, this method prevents having a curve with saturated values at the maximum of a supposed limited traditional scale. The result from the module (2) is a signal (a) relativised at the maximum value thereof with the aim of normalising all the migraine recordings of the patient. The synthesised symptomatic pain curve (a) is described by the parameters $\{(\mu1, \sigma1), (\mu2, \sigma2)\}$ that make up the semi-Gaussian ones. The module (2), as well as the collection of pain data (d2) is only performed not in real time, for creating the models.

**B) Training and validation step**

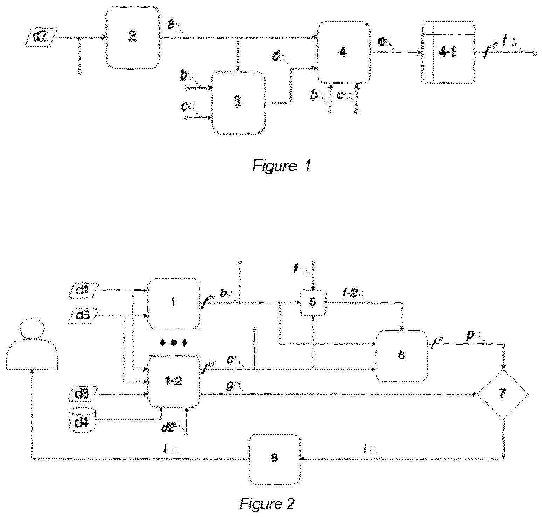

This step is performed offline, not in real time. During the training step, the patient is monitored for a period of time wherein a number of migraines (T) that is sufficient to train the models is recorded. These modelling algorithms generate a prediction curve ($\hat{y}$) for each migraine starting from the multisource data collected. During training, the curve resulting from the models (signal d in Figure 1) is compared to the curve generated (a) based on the subjective pain sensation (d2). The metrics used to evaluate the validity of the prediction models generated is the fit, defined as:

**Equation 1:**

$$fit = 100\left(1 - \frac{\|y - \hat{y}\|}{\|y - average(y)\|}\right),$$

where y is the calculated symptomatic curve (signal a) marked by the patient, and $\hat{y}$ is the prediction.

**a) Training module (3)**

For each one of the recorded migraines a model is trained. The migraine models are created with the module (3). In order to create the migraine models, the repaired biometric signals (b[1]), those derived with temporal variation (c[1]) and the synthetic pain curve (a) are used. In the training the search for the prediction horizon that best adapts to each patient will be performed. The method evaluates different modelling techniques or algorithms; unlike the patent proposed by Geatz M. and Rolger R., which is only based on Artificial Neural Networks, our proposal and prior work have demonstrated that different modelling techniques must be evaluated for each patient, and the best of them all must be chosen. The models are or are not linear functions and have the primary biometric signals, secondary biometric signals or one of the calculated features as input variables (all of them being included in the variable sets b[1] and c[1]), and the result of which is a prediction (d) of the evolution of the migraine attack at a given horizon.

## Equation 2:

$$\hat{y}(t) = f(\{h1(t), h2(t), \ldots, hs1(t), \ldots, hs3(t), hd1(t), \ldots hdH(t)\})$$

Equation 2 represents the predicted symptomatic curve $\hat{y}(t)$ as a function of the primary (h), secondary (hs) or derived (hd) biometric variables that vary in time, all of them being contained in the signal (b[1]).

Some modelling diagrams or valid algorithms that are used successfully are: State-Space Equations, Artificial Neural Networks or Genetic Programming. The following is an example of a generic equation (Equation 3) from a state-space system of order nx:

## Equation 3:

$$x[k+1] = Ax[k] + Bu[k] + w[k]$$
$$y[k] = Cx[k] + Du[k] + v[k]$$

Where y[k] is the output at the moment k, which depends on the current state, x[k], and a matrix, C. The order of the system, nx, is determined by the dimension of x[k]. The variable v[k] is the innovation or unexplained portion that is added to the prediction, ideally white noise uncorrelated with the input variables, u[k]. The output can also depend on the inputs at that same moment, through the vector D, which is weighted by the input variable vector, u[k]. The following state of the method is defined by the equality of x[k+1]. The dependency between states is weighted by the state transition matrix (A), while the weight matrix of the variables (B) sets the dependence on the entry variables, u[k]. w[k] is, ideally, white noise uncorrelated with the current state, and is the unexplained portion or error that occurs when passing from one state to another.

This invention proposes the creation of different models for different input variable sets with the aim of subsequently developing a hierarchy for selecting models according to the sensors or variables available. This hierarchy lies underneath the idea of creating a robust method, which is tolerant to errors and to partial sensor failure. At the end of the Training step, $M_d$ different trained models are had and for each different combination of biometric input variables, with d = 1,2,...,N. N can be one or several migraines. The amount of models $M_d$ created depends on the type of algorithm selected and the parameters thereof.

Another set of models (independent from the biometric variables) is also trained with the use of the environmental variables (sets b[2] and c[2]). The prediction models based on environmental variables are coarse-grained models, and they have less temporal definition than the predictions based on biometric variables; for this reason, these predictions will only be used in the decision module (7).

**b) Validation module and selection of models (4 and 4-1).**

The module (4) searches for the best models to predict the migraines of the patient by using Cross Validation techniques. Each model $M_{d,i}$ (obtained from the set $M_d$), with $i$ = 1,...,$d$, is validated by predicting the remaining migraines not used in the Nth combination that created said model. The validations are performed in a prediction horizon that does not necessarily coincide with that which trained the model, otherwise it will search for the one further away with better prediction results.

The validation implies checking how each of these models adjusts (according to the fit) to the prediction of other migraines. The results of this module (e) is a set of models that will be ranked and stored in the module (4-1) in order to give as a final result a batch or set of the $M_{best}$ models (signal f) that best predict the migraines of the patient in question, if $M_d > 1$. The selection criteria are: the prediction horizon, the robustness of the model when faced with sensor failure or saturation of the signal and the complexity or order of the model. The selection of a set of models, instead of only one, gives greater stability to the method and mitigates the use of models with overfitting.

For each different combination of input biometric variables (b[1] and c[1]), a batch of models is finally had. The same process is followed for the environmental variables (b[2] and c[2]).

**C) Step of prediction in real time. Expert system**

This step is performed in a loop in real time and the data can continue to be sent to the servers, or not. If it is sent, a control in real time of the state of the patient can be had, and in the case of false negatives, there would be records to be able to confirm how said errors occurred. With the batch of models of each patient the prediction of the migraine attack is performed by using the primary, secondary and/or derived biometric variables. The steps in which the prediction is performed are detailed below.

**a) System for Selecting Models Depending on the Sensors (SMDS²) (5)**

This model provides robustness to the method by adaptation, and not by redundancy or a high amount of trained models. The migraines of each patient can be defined by a set of variables ($v_1, v_1 = b \cup c$) different from that of another patient; furthermore, in an outpatient monitoring context, the error of the sensors due to unavailability or saturation is a recurring problem. In this scenario, a hierarchy or criteria is defined for selecting models is defined per patient and depends on the input variables that are necessary and available. This adaptation is what makes the method robust.
After the validation and selection of the models of a patient the input variables that best define their migraines then are known. In this case, the models that are chosen and have that combination of variables as an input are used ($\{M_{best}, v_1\}$). If, due to an error or breakage, one of the necessary sensors is not available, the next set of models with better fit and that do not depend on said sensor is used ($\{M_{best}, v_2\}$).
This module, therefore, indicates the set of models of the module (4-1) to be used (6) both for the biometric variables and the environmental variables.

**b) Prediction and linear combination of models (6)**

By using the models obtained from (5) and with the biometric signals (b[1] and c[1]), the prediction of the migraine will be performed. The prediction horizon is the best one that can be obtained with each model. Each one of the models from the batch (5) performs a prediction on the data from the necessary biometric variables (b[1] and c[1]). In total $M_{best}$ predictions are had. The final result is a linear combination (p) of all these predictions. Since prediction models are also trained through the environmental variables, they are also used to make predictions, for which reason the signal (p) is the union of the average predictions obtained with the biometric inputs (p[1]) and the environmental inputs (p[1]). Thus, $p = p[1] \cup p[2]$.

**c) Correction and fit of the prediction (7)**

With the aim of obtaining the best predictions, this module performs a correction of the prediction (p[1]) through the biometric variables (b[1] and c[1]), removing false data points from said signal. The false data points are detected by defining level and time thresholds.
Furthermore, in this module decisions are taken on the prediction through another prediction (p[2]) made with the second set of variables (g) comprising: the environmental variables (b[2] and c[2]), the information obtained with the data mining and fuzzy logic techniques on the features of the biometric and environmental variables, and the activity and clinical data of the patient. These decisions are weightings or weights for the prediction. The result is double, on one hand a prediction curve for a given horizon, and on the other a warning signal of the beginning of pain with a probability of occurrence.
The environmental variables generate longer-term predictions with less precision, but they serve as contrast to the prediction performed with the biometric variables. These contrasts or decisions are carried out with fuzzy logic techniques trained beforehand for the patient in the module (1-2). This module presents the corrected, fitted and contrasted prediction (p) at the output (i) thereof. It returns, apart from the prediction, a decision of at what moment and with which probability the pain will occur.

**d) Actuator (8)**

The actuator is the last module of the method, it is a man-machine interface (8), and it re-supplies the patient with the prediction that was made and corrected (i). In this manner the patient can take the medication with enough time for it to take effect and have complete effectiveness before the pain appears. The prediction also arrives at the devices for monitoring biometric and local environmental variables so that they can evaluate whether to stop, or not, the monitoring during a known period of time. The interface will be the mobile application that gathers information from the activity of the patient, since the patient will have it with them at all times.
The novelty of this invention is rooted in the following technical characteristics:

- Personalised prediction of migraine attacks by using hemodynamic variables of the patient and of the cerebral electrical activity. Furthermore, using climatological and environmental variables, clinical data and information relating to the activity of the patient as support for the prediction.
- Personalised training of models for each patient with automatic variable selection.
- Creation of a hierarchical set of models. Development of an automatic selection method of the hierarchical model depending on the available input variables. Robust and error-tolerant method maintaining a certain prediction horizon.
- Creation of a module to help the prediction and repair of models that improves the validity of the prediction.
- Interface or warning system (actuator) for the effective intake of the medicinal product that stops the pain of the migraine before it appears.

**BRIEF DESCRIPTION OF THE DRAWINGS**

The following drawings illustrate the preceding description:

Figure 1 shows the diagram of the creation of models not in real time. This diagram is executed in the training step and gives rise to the models (f) that will predict the migraines of each patient. The models are created in the module (3) through the biometric variables (b[1] and c[1]) and the synthesised pain signal (a). Different models are created for each different combination of variables. The models are validated in the module (4); the best models (f) will be chosen from these models in hierarchical order in the module (4). In turn, the module (3) also trains prediction models based on environmental variables (b[2] and c[2]); moreover, for each different combination of these variables different models are created and the best ones are chosen.

Figure 2 shows the diagram of modelling and prediction in real time of the migraine prediction method through the hemodynamic variables of the patient and the cerebral electrical activity (d1), and with support from local and overall environmental variables (d5). Said variables are pre-processed and synchronised in the pre-processing module (1). In this prediction step (in a loop) the suitable models are chosen for the patient and depending on the variables available in the module (5) and the prediction (p[1]) is performed through the linear combination of models of biometric variables in the module (6). The prediction arrives at the decision module (7) where the false events are removed and, with help from the prediction of the environmental variables (p[2]) and the result from Machine Learning techniques (g), it is decided if a migraine (i) has been detected or not. The decision arrives at the actuator (8), which warns the patient in order to move up the ingestion of the medicinal product and prevent the pain.

**EMBODIMENT OF THE INVENTION**

The monitoring implies the recording of a sufficient number of migraines for the training step. In order to train the modelling algorithms, it is considered that the training can be sufficient as of 10 migraine episodes (the monitoring time generally oscillates between 4 and 6 weeks). One possible way of proceeding to acquire data is described below in an indicative and non-exhaustive manner.

The monitoring of the biometric variables (d1) is carried out with commercial outpatient monitoring devices. The ECG sensor can have as many derivations as desired, but having only three electrodes is enough to extract the HR (hs1); in this case, they will be placed on the precordial horizontal plane in derivations V3, V4 and V5. The EDA sensor (h1) placed in the arm acts to measure the relative variations in perspiration; like the superficial temperature sensor (h2), placed as close as possible to the armpit. One way of acquiring the SpO2 (h3) and the PPG (h4) is by means of the use of an oximetry clip placed on a finger. The EEG electrodes will be placed on the occipital area, in the reference points OZ, O1 and O2 (according to the international system 10-20). The PTT will be calculated through the ECG and PPG signals and by applying one of the bibliographical methods (Yoon Y, Cho JungH, Yoon Gilwon, Non-constrained Blood Pressure Monitoring Using ECG and PPG for Personal Healthcare. 2009; 33(4):261-266). The HR can be calculated in intervals of 20 seconds with a 10 second overlap. The qEEG is the energy of the Alpha, Beta, Gamma, Delta and Theta bands in 20 second intervals without overlap.

At the same time that the biometric variables (d1) are recorded, the local and overall environmental variables (d5) are recorded. The overall environmental variables are taken from the geographic area corresponding to the location of the patient, and a national weather service can be used. The local environmental variables are monitored through a weather station that is always near the patient. The good synchronisation of all the data must be taken into account; to do so, a smartphone can be used to capture all the weather data. The subjective sensation of the pain (d2) is recorded through a mobile application. The patient indicates the beginning and end of the pain, as well as the subjective evolution thereof. With the same mobile application, the activity of the patient (d3) is recorded, and furthermore, there is knowledge of some of their clinical data (d4) relevant for the study, such as weight, age, gender or diseases related to migraines. All the data (d1-d5) collected during the training step is pre-processed (1 and 1-2) and used to train migraine models in (3).

Both the training of the models, and the validation (4), are performed not in real time in high-capacity computing equipment. The result of the training step is a set of models that is different for each possible combination of variables. (f). The models are trained by means of supervised techniques, where the inputs are the processed variables, and the output to be fitted is the subjective pain of the patient pre-processed in (2). Models will be trained for all the possible combinations of input variables. In validation, the best ones will be ranked and chosen to be used in the prediction step in real time.

In the prediction step in real time, the system for selecting models depending on the sensors (5) is used to select the variables of interest of each patient and the hierarchical set of models (f-2) depending on the sensors available. To do so, the state of the sensors must be known at all times, and if there are any that are not available, the models will be changed. Once the chosen models are had, these are applied one by one to the input variables, giving rise to a set of predictions; the final prediction (p) is calculated in (6) like the linear combination of said predictions. The horizon in which the prediction is performed will depend on the quality of the model obtained, for example 30 minutes. The module for correction and fit of the prediction (7) removes the false prediction points according to criteria of duration and degree of detection; thus, possible false alarms are able to be eliminated. Furthermore, decision criteria (g) are applied in order to weight the answer. The prediction obtained from the biometric variables is weighted by the decision criteria. These weightings are the result of the fuzzy logic algorithms that, based on the knowledge of the environmental variables and of the activity of the patient, regulate the prediction, for example lessening or increasing the levels thereof. Finally, the prediction (i) is transmitted to the patient through the actuator module (8, the mobile phone) so that it can move up the ingestion of the medicinal product against the migraine pain, before it begins.

The retraining of the models can be carried out automatically in a transition period in which it is still in the real-time prediction step, but the set of models of the patient starts to be updated. The retraining will be necessary when the patient finds that the predictions are no longer correct or a clinical evaluation considers it appropriate. In real time, the patients will not mark the evolution of their pain (d2) for which reason the only record of errors in the prediction that can be had will be the one of the false positives (migraines that were not detected).

The hardware of the monitoring devices must have enough computational capacity to be able to sample the variables at the sampling rate required and send the data wirelessly. The capacity to stop monitoring when they are aware of the state of the prediction must also be supported by the hardware and the firmware of the devices.

The present invention has application in the field of medical devices for the early alert of migraine pain. The network of electronic outpatient health monitoring devices approved for medical use is increasingly widespread and established; furthermore, the portability thereof and the duration of the batteries thereof keeps increasing. The machine-man interface for alerting patients is performed through a smartphone terminal, which is common today. For all of this, the present invention can have an immediate use in monitoring migraine patients in order to predict their attacks.

This invention enables those suffering from migraines to perform the earlier ingestion of the medication against migraine pain so that it has a complete effect and they are able to thus prevent the painful phase of the migraine. The use of this method increases the quality of life of the patients, as well as reduces the direct and indirect costs that the disease causes worldwide.

**Claims**

1. A method for determining the degree of activation of the trigeminovascular system based on the monitoring of biometric variables comprising the execution of the following steps:

   a. Monitoring of biometric and environmental variables and subjective degree of activation of the trigeminovascular system for training models, structured in the following sub-steps:

   i. Pre-processing of the signals by means of statistical techniques based on the knowledge of the history of each signal, the following values thereof and the distribution thereof (average and standard deviation, among others);
   ii. Objectification of the subjective measurement of the degree of activation of the trigeminovascular system by means of normalisation of levels and bilateral Gaussian fit with reference to the maximum level recorded;

   b. Amplification of the set of significant variables for training the models through the following sub-steps:

   i. Generation of secondary signals
   ii. Generation and selection of features of the signals acquired and of the secondary signals;

   c. Estimation of the degree of activation of the trigeminovascular system starting from the monitored variables, the secondary variables and the features generated according to the following sub-steps:

i. Generation of groups of variables, combinations of, at least, two of them;

ii. Training of the models, one for each group of input variables and with reference to the objective degree of activation;

iii. Selection of the models according to the input variables used and the degree of similarity of the signal that they produce ($\hat{y}$) with the degree of objectivity (y) expressed in the following formula:

$$fit = 100 \left( 1 - \frac{||y - \hat{y}||}{||y - average(y)||} \right)$$

iv. Starting from the available variables and the model chosen, a first estimation of the degree of activation of the trigeminovascular system is obtained as an output thereof;

d. Reduction of the estimation error of the degree of activation of the trigeminovascular system by means of the use of a second set of variables, which can use expert knowledge strategies, such as data mining and/or fuzzy logic, for the correction and fit of the estimated degree of activation of the trigeminovascular system.

2. The method according to claim 1 for monitoring hemodynamic biometric variables, occipital electroencephalogram, climatological signals from the surroundings and environmental signals for the sending thereof to a cloud storage platform to be processed.

3. The method according to the preceding claims, wherein the data processing is structured in the following sub-steps:

a. Synchronisation of the different signals with time marks of all the data;

b. Elimination of out-of-range data and filtering of the signals;

c. Application of techniques for automatic signal regeneration based on the statistical behaviour of the signal;

d. Decimating the signals to reduce the amount of input data for the models of claim 1;

e. Objectification of the degree of activation of the trigeminovascular system by means of a non-limited level scale and a continuous Gaussian fit mechanism with discreet subjective values of the degree of activation of the trigeminovascular system.

4. The method according to the preceding claims, **characterised by** the generation of secondary signals and characteristic features of the signals that is developed in the following sub-steps:

a. Calculation of the heart rate (HR) by counting the number of events of the ECG signal in time windows of 20 seconds with 10 seconds of overlap by defining wait times between peaks and level decision criteria for the failure to detect false positives;

b. Calculation of the pulse transit time (PTT) for determining the arterial pressure by means of regression functions calculated with the peaks detected from the PPG and ECG signal;

c. Calculation of the qEEG signal through the calculation of the energy of the band pass filtering without overlap of the EEG signal.

5. The method according to the preceding claims, **characterised by** a System for Selecting Models Depending on the Sensors (SMDS²) consisting of the precedence of models for estimating the degree of activation of the trigeminovascular system which can be performed by means of a mechanism based on statistical confidence.

6. The method according to the preceding claims, **characterised by** the linear combination of the set of models set out in Claim 5.

7. The method according to the preceding claims, wherein the reduction of the estimation error in Claim 1 is developed in three sub-steps;

a. Detection and elimination of events by defining a threshold for which the events that do not determine a degree of activation of the trigeminovascular system with an index greater than 50% with respect to the maximum will be eliminated;

b. Detection and elimination of events based on time by defining a temporal threshold of 60 minutes, wherein the events that exceed the level threshold but have a duration less than the time threshold will be eliminated; while the events that are at a shorter distance than this threshold of another event will be considered the same;

c. Application of expert knowledge techniques, such as fuzzy logic algorithms, in order to grant degree of confidence to the activation events of the trigeminovascular system able to re-feed the signal to the monitoring signal.

8. The method according to claim 1, wherein the monitoring of biometric variables obtained by means of sensors will be developed in the following sub-steps:

a. The detection of the state will be able to be carried out by means of a decision taken on the statistics of the data recorded in previous moments;
b. If a sensor is not available, the models that include variables dependent on it will not be chosen.

9. The method according to claim 1 based on mobile equipment that communicate the information to the monitoring devices.

Figure 1

Figure 2

# INTERNATIONAL SEARCH REPORT

| International application No. |
| --- |
| PCT/ES2017/070004 |

## A. CLASSIFICATION OF SUBJECT MATTER

*G06F19/00* (2011.01)
*G08B21/02* (2006.01)
According to International Patent Classification (IPC) or to both national classification and IPC

## B. FIELDS SEARCHED

Minimum documentation searched (classification system followed by classification symbols)
G06F, G08B

Documentation searched other than minimum documentation to the extent that such documents are included in the fields searched

Electronic data base consulted during the international search (name of data base and, where practicable, search terms used)

EPODOC, INVENES, WPI, Google Scholar

## C. DOCUMENTS CONSIDERED TO BE RELEVANT

| Category* | Citation of document, with indication, where appropriate, of the relevant passages | Relevant to claim No. |
| --- | --- | --- |
| Y<br>A | PAGÁN, J. et al.. Robust and Accurate Modeling Approaches for Migraine Per-Patient Prediction from Ambulatory Data. Sensors, 30/06/2015, Vol. 15, Pages 15419-15442 [on line][retrieved 11/04/2017]. Retrieved from Internet <URL: http://www.mdpi.com/1424-8220/15/7/15419>, ISSN 1424-8220, <DOI: 10.3390/s150715419> p. 15419; Sec. Abstract, p. 15420; Sec. 1; p. 15423; Sec. 3.1; p. 15424; Sec. 3.2; p. 15425; Sec. 3.3; p. 15426; Sec. 4; p. 15426; Sec. 4.1; p. 15426; Sec. 4.1.3; p. 15427; Sec. 4.1.4; p. 15429; Sec. 4.1.5; Fig. 3; p. 15433; Sec. 5.3.1; Ec. (1); Fig. 1, Fig. 2, Fig. 3 | 1 - 3, 5 - 9<br>4 |
| Y<br>A | US 2010253509 A1 (FU YONGJI   ET AL.) 07/10/2010, Abstract; [0004]-[0024]; Fig. 5 | 1 - 3, 5 - 9<br>4 |

☒ Further documents are listed in the continuation of Box C.       ☒ See patent family annex.

| | |
| --- | --- |
| *    Special categories of cited documents: | "T"   later document published after the international filing date or priority date and not in conflict with the application but cited to understand the principle or theory underlying the invention |
| "A"  document defining the general state of the art which is not considered to be of particular relevance. | |
| "E"  earlier document but published on or after the international filing date | |
| "L"  document which may throw doubts on priority claim(s) or which is cited to establish the publication date of another citation or other special reason (as specified) | "X"   document of particular relevance; the claimed invention cannot be considered novel or cannot be considered to involve an inventive step when the document is taken alone |
| "O"  document referring to an oral disclosure use, exhibition, or other means. | "Y"   document of particular relevance; the claimed invention cannot be considered to involve an inventive step when the document is combined with one or more other documents , such combination being obvious to a person skilled in the art |
| "P"  document published prior to the international filing date but later than the priority date claimed | "&"   document member of the same patent family |

| Date of the actual completion of the international search | Date of mailing of the international search report |
| --- | --- |
| 11/04/2017 | **(12/04/2017)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>J. Carbonell Olivares<br><br><br>Telephone No. 91 3496837 |

Form PCT/ISA/210 (second sheet) (January 2015)

## INTERNATIONAL SEARCH REPORT

| International application No. |
| --- |
| PCT/ES2017/070004 |

C (continuation).                    DOCUMENTS CONSIDERED TO BE RELEVANT

| Category * | Citation of documents, with indication, where appropriate, of the relevant passages | Relevant to claim No. |
| --- | --- | --- |
| A | WO 2013177688 A1 (NEXT INTEGRATIVE MIND LIFE SCIENCES HOLDING INC) 05/12/2013,<br>[0085], [0087], [0145], [0147]; Fig. 1C, Fig. 1D | 1 - 9 |
| A | WO 2008092133 A2 (NEUROVISTA CORP ET AL.) 31/07/2008,<br>Sec. "Summary of the invention"; Fig. 1 | 1 - 9 |
| A | US 2015324544 A1 (MASLOWSKI ERIC ET AL.) 12/11/2015,<br>[0006]-[0009]; Fig. 2A, Fig. 4A | 1 - 9 |

Form PCT/ISA/210 (continuation of second sheet) (January 2015)

## INTERNATIONAL SEARCH REPORT

Information on patent family members

International application No.

PCT/ES2017/070004

| Patent document cited in the search report | Publication date | Patent family member(s) | Publication date |
|---|---|---|---|
| US2010253509 A1 | 07.10.2010 | JP2012522969 A<br>JP5655005B B2<br>CN102388323 A<br>WO2010113650 A1<br>EP2414869 A1<br>EP2414869 A4<br>US8085145 B2 | 27.09.2012<br>14.01.2015<br>21.03.2012<br>07.10.2010<br>08.02.2012<br>12.09.2012<br>27.12.2011 |
| WO2013177688 A1 | 05.12.2013 | US2015339363 A1<br>CA2874932 A1 | 26.11.2015<br>05.12.2013 |
| WO2008092133 A2 | 31.07.2008 | US2011213222 A1<br>US2011172554 A1<br>EP2124734 A2<br>US2008183097 A1 | 01.09.2011<br>14.07.2011<br>02.12.2009<br>31.07.2008 |
| US2015324544 A1 | 12.11.2015 | NONE | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03063684 A, Geatz M, Roiger R.
- ES 2334733
- US 2011270095 A
- TW 201023087
- US 8123683 B

**Non-patent literature cited in the description**

- **LINDE M ; RASTENYTE D ; RUIZ DE LA TORRE E et al.** The cost of headache disorders in Europe: The Eurolight project. *European journal of neurology: the official journal of the European Federation of Neurological Societies,* 2012, vol. 19 (5), 703-711
- **WAELKENS J.** Dopamine blockade with domperidone: Bridge between prophylactic and abortive treatment of migraine?. *A dose-finding study. Cephalalgia,* 1984, vol. 4 (2), 85-90
- **LUCIANI R ; CARTER D ; MANNIX L ; HEMPHILL M ; DIAMOND M ; CADY R.** Prevention of migraine during prodrome with naratriptan. *Cephalalgia,* 2000, vol. 20 (2), 122-126
- **BECKER WJ.** The premonitory phase of migraine and migraine management. *Cephalalgia,* 2013, vol. 33 (13), 1117-1121
- **ROSSI P ; AMBROSINI A ; BUZZI MG.** Prodromes and predictors of migraine attack. *Funct Neurol.,* 2005, vol. 20 (4), 185-191
- **GIFFIN NJ ; RUGGIERO L ; LIPTON RB et al.** Premonitory symptoms in migraine: An electronic diary study. *Neurology,* 2003, vol. 60 (6), 935-940
- **GASS JJ ; GLAROS AG.** Autonomic dysregulation in headache patients. *Appl Psychophysiol Biofeedback,* 2013, vol. 38 (4), 257-263
- **MOSEK A ; NOVAK V ; OPFER-GEHRKING TL ; SWANSON JW ; LOW PA.** Autonomic dysfunction in migraineurs. *Headache: The Journal of Head and Face Pain,* 1999, vol. 39 (2), 108-117
- **SANYA E ; BROWN C ; WILMOWSKY C ; NEUNDÖRFER B ; HILZ M.** Impairment of parasympathetic baroreflex responses in migraine patients. *Acta Neurol Scand.,* 2005, vol. 111 (2), 102-107
- **BENJELLOUN H ; BIROUK N ; SLAOUI I et al.** Autonomic profile of patients with migraine. *Neurophysiol Clin.,* 2005, vol. 35 (4), 127-134
- **DURU M ; MELEK I ; SEYFELI E et al.** QTc dispersion and p-wave dispersion during migraine attacks. *Cephalalgia,* 2006, vol. 26 (6), 672-677
- **ORDÁS CM ; CUADRADO ML ; RODRIGUEZ-CAMBRON AB ; CASAS-LIMÓN J ; DEL PRADO N ; PORTA-ETESSAM J.** Increase in body temperature during migraine attacks. *Pain Medicine.,* 2013, vol. 14 (8), 1260-1264
- **PORTA-ETESSAM J ; CUADRADO ML ; RODRIGUEZ-GOMEZ O ; VALENCIA C ; GARCIA-PTACEK S.** Hypothermia during migraine attacks. *Cephalalgia,* 2010, vol. 30 (11), 1406-1407
- **BJØRK M ; SAND T.** Quantitative EEG power and asymmetry increase 36 h before a migraine attack. *Cephalalgia,* 2008, vol. 28 (9), 960-968
- **YOON Y ; CHO JUNGH ; YOON GILWON.** *Non-constrained Blood Pressure Monitoring Using ECG and PPG for Personal Healthcare,* 2009, vol. 33 (4), 261-266